# EUROPÄISCHE PATENTSCHRIFT

(11) **EP 1 723 343 B1**
(45) Veröffentlichungstag und Bekanntmachung des Hinweises auf die Patenterteilung: **15.08.2007**
(21) Anmeldenummer: 04718898.2
(22) Anmeldetag: 10.03.2004
(51) Int. Cl.: F16B 7/04, A61B 17/64

(54) **VORRICHTUNG ZUR GEGENSEITIGEN POSITIONIERUNG VON LONGITUDINALEN BAUELEMENTEN**
DEVICE FOR MUTUAL POSITIONING OF LONGITUDINAL BUILDING COMPONENTS
DISPOSITIF SERVANT AU POSITIONNEMENT MUTUEL DE COMPOSANTS LONGITUDINAUX

(43) Veröffentlichungstag der Anmeldung: 22.11.2006
(73) Patentinhaber: Synthes GmbH, 4436 Oberdorf (CH)
(72) Erfinder: OESCH, Marc, CH-2544 Bettlach (CH); LANZ, Andreas, CH-3270 Aarberg (CH)
(74) Vertreter: Lusuardi, Werther
(86) Internationale Anmeldenummer: PCT/CH2004/000139
(87) Internationale Veröffentlichungsnummer: WO 2005/085658

(56) Entgegenhaltungen:
- DE-A- 4 431 278
- DE-U- 9 106 772
- US-A- 3 651 449
- US-A- 4 920 959
- US-A1- 2002 151 892
- US-B1- 6 342 054

## Beschreibung

Die Erfindung bezieht sich auf eine Vorrichtung zur gegenseitigen Positionierung von longitudinalen Bauelementen gemäss dem Oberbegriff des Patentanspruchs 1.

Solche Vorrichtungen eignen sich insbesondere als Befestigungsmittel für Gestelle, Stative, Stangenkonstruktionen, dreidimensionale Rahmenkonstruktionen sowie für chirurgische Fixationsvorrichtungen.

Aus der EP-B 0 700 664 ist eine Vorrichtung bekannt, bei welcher vier separate, auf einer Welle gelagerte Backen zwei Öffnungen definieren, in welche Befestigungsstangen oder Steckverbinder gegen die Kraft einer zwischen den Backenpaaren positionierten Spiralfeder eingeführt werden können. Nachteilig bei dieser bekannten Vorrichtung ist die grosse Zahl von Einzelteilen und ihre geringe Flexibilität, bzw. Anpassbarkeit an die Geometrie der in die Öffnungen einzuführenden Bauteile.

Aus der US 5,727,899 DOBROVOLNY ist eine Vorrichtung mit zwei Klammern bekannt, welche allerdings nicht für einen Fixateur externe vorgesehen ist. Diese bekannte Vorrichtung ermöglicht zwar ein laterales Einklipsen eines Längsträgers aber nur in eine der beiden Klammem. Dieses Einklipsen geschieht aber nicht durch elastische Deformation der Klammer und auch nicht gegen den Druck einer zwischen den beiden Klammern angeordneten Feder, sondern benötigt dazu eine separate Feder, welche in der Klammeröffnung angeordnet ist. Die als Drehgelenke wirkenden Übergänge (Fulcri) sind zwischen den Klammeröffnungen und den Befestigungsenden angebracht, so dass bei einem Aufspreizen der Befestigungsenden - mittels des Hebels 90 - sich die Klammeröffnungen schliessen und die darin befindlichen Längsträger klemmen. Zu diesem Zweck haben die Klammern eine H-Form. Die Fulcri befinden sich dabei zwischen den Klammeröffnungen und den Befestigungsenden. Weitere Nachteile dieser bekannten Vorrichtung sind die folgenden:
- grosse Anzahl von Einzelteilen
- komplizierte und aufwändige Montage
- komplizierte und schwierige Reinigung es können nur Stäbe mit einem bestimmten Durchmessser aufgenommen werden.

Aus der US 2002/0151892 WALULIK ET AL. ist eine gattungsgemässe Vorrichtung bekannt. Eine erste Ausführungsform betrifft zweistückige Klammern, eine zweite Ausführungsform einstückige, elastisch federnde Klammern, welche derart ausgebildet sind, dass sie völlig koaxial zur zentralen Welle zusammendrückbar sind. Zu diesem Zweck sind die Klammern ohne Spiel auf der zentralen Welle positioniert.

Hier will die Erfindung Abhilfe schaffen. Der Erfindung liegt die Aufgabe zugrunde, eine Vorrichtung zur gegenseitigen Positionierung von longitudinalen Bauelementen zu schaffen, welche einfach und aus einem Minimum von Einzelteilen konstruiert ist und die eine grosse Flexibilität bezüglich der aufzunehmenden und gegeneinander zu positionierenden Bauelemente aufweist.

Die Erfindung löst die gestellte Aufgabe mit einer Vorrichtung, welche die Merkmale des Anspruchs 1 aufweist und mit einer Klammer für eine solche Vorrichtung zur gegenseitigen Positionierung von longitudinalen Bauelementen, welche die Merkmale des Anspruchs 15 aufweist.

Die durch die Erfindung erreichten Vorteile sind im wesentlichen darin zu sehen, dass dank der S-förmigen Ausgestaltung mindestens einer der beiden Klammern einerseits die umständlichen Einzelbacken entfallen und anderseits keine separate Spiralfeder, die sich zwischen den Backen verklemmen kann, notwendig ist. Zudem erlaubt die erfindungsgemässe Konstruktion im Vergleich zum Stand der Technik eine grössere Flexibilität bezüglich der in die Klammeröffnungen aufzunehmenden Bauelemente, deren Durchmesser innerhalb eines grösseren Bereiches variieren können. Damit ist es möglich mit einer einzigen Vorrichtung eine grosse Anzahl von verschieden dimensionierten Bauelementen gegeneinander zu positionieren, währenddem beim Stand der Technik Bauelement und Befestigungsvorrichtung auf einander abgestimmt sein müssen, so dass eine Vielzahl von verschieden dimensionierten Befestigungsvorrichtungen notwendig sind, um alle Anwendungsfälle bewältigen zu können.

Bei einer besonderen Ausführungsform weist mindesten eine der beiden Klammern eine oder mehrere parallel zur Rotationsachse verlaufende Bohrungen auf, in welchen Arretierstifte parallel zur Rotationsachse bewegbar sind. Diese Arretierstifte dienen dazu, dass sich die Klammeröffnungen nicht so weit schliessen können, dass dadurch die diametral gegenüberliegende Klammeröffnung wieder geöffnet würde.

Bei einer weiteren Ausführungsform weist die Bohrung in den beiden Klammern mindestens teilweise einen grösseren Durchmesser als die Welle auf. Dadurch können einzelne Klammerschenkel relativ zur Welle abgekippt werden, um dadurch die Klammeröffnung zu verengen oder zu erweitern.

Bei einer weiteren Ausführungsform sind die Klammerschenkel derart miteinander verbunden, dass ein zangenähnliches Öffnen und Schliessen der Klammerschenkel um einen Drehpunkt realisierbar ist.

Vorzugsweise ist die Klammer einstückig ausgebildet. Die Vorteile gegenüber einer zweistückigen Ausführung liegen darin, dass weniger Einzelteile erforderlich sind und dass kein separates Federelement nötig ist, da die Klammer selbstfedernd ist.

Bei einer weiteren Ausführungsform weist die Klammeröffnung eine Verengung auf. Dadurch kann man in einem ersten Schritt mit einem longitudinalen Bauelement, z.B. einem Stab, einem Längsträger oder einer Schraube die Aussen verengte Klammeröffnung gegen die Elastizität der Klammer aufweiten, so dass das Bauelement seitlich in die Klammeröffnung eingeführt werden kann. Die im Inneren erweiterte Klammeröffnung schliesst sich dann dank der Elastizität der Klammer automatisch um das eingeführte Bauelement herum und garantiert eine primäre Vorfixierung des Bauelementes in der Klammeröffnung. In einem zweiten Schritt können dann mittels der Blockiermittel beide Klammern gleichzeitig und mit den darin vorfixierten Bauelementen definitiv gegeneinander fixiert werden.

Bei einer weiteren Ausführungsform weist die Klammeröffnung nach Aussen eine Erweiterung auf. Damit lassen sich die longitudinalen Bauelemente leichter gegen die Elastizität der Klammer in die Klammeröffnung eindrücken.

Bei einer weiteren Ausführungsform sind die Klammeröffnungen kanalartig ausgebildet und weisen eine Kanalachse auf. Mindestens eine der Klammeröffnungen weist dabei eine zu ihrer Kanalachse orthogonale Querschnittsfläche auf, deren Begrenzung an den angrenzenden Klammerschenkeln mehrere hintereinander angeordnete Kreisbogen mit unterschiedlichen Durchmessern aufweist. Der Vorteil dieser Ausführung liegt darin, dass in diesen Klammeröffnungen Stäbe mit verschiedenen Aussendurchmessern festgeklemmt werden können.

Bei einer weiteren Ausführungsform umfassen die einander benachbarten Klammerschenkel der beiden Klammern ineinander eingreifend eine zur Rotationsachse koaxiale konische Erhebung, respektive konische Vertiefung. Dadurch werden beim Blockieren der Vorrichtung die beiden konischen Elemente ineinander gepresst, so dass durch die daraus entstehende Verkeilung eine Rotation um die Rotationsachse der beiden Klammern relativ zueinander verhindert werden kann.
Vorzugsweise sind die Oberflächen der konischen Erhebung und/oder der konischen Vertiefung aufgerauht, um damit eine Verstärkung der die relative Rotation der beiden Klammern verhindernden Verkeilung der beiden konischen Elemente zu erhalten. Anstelle der Aufrauhungen sind auch ineinander in Eingriff bringbare, stirnseitige Verzahnungen oder konische Verzahnungen möglich.

Bei einer weiteren Ausführungsform weist die Welle in der Bohrung ein Spiel auf, so dass eine Schrägstellung der Klammerschenkel gegenüber der Rotationsachse bei der Klemmung von Stäben durch die Welle nicht verhindert wird.

Bei einer weiteren Ausführungsform umfasst die Bohrung an ihren aussenstehenden Austritten Bohrungssegmente, welche je eine zur Rotationsachse konzentrische, konkav kugelzonenartige Oberfläche aufweist. Vorzugsweise weist die Welle einen, einen grösseren Durchmesser aufweisenden Kopf mit einer zur konkav kugelzonenförmigen Oberfläche der Bohrungssegmente komplementär konvex kugelzonenförmige Oberfläche auf.

Bei einer weiteren Ausführungsform weisen die Blockiermittel ein Zwischenstück mit einer zur konkav kugelzonenförmigen Oberfläche der Bohrungssegmente komplementär konvex kugelzonenförmigen Oberfläche auf. Damit können Schrägstellungen zwischen der Welle und der Bohrung kompensiert werden.

Die Erfindung und Weiterbildungen der Erfindung werden im folgenden anhand der teilweise schematischen Darstellungen mehrerer Ausführungsbeispiele noch näher erläutert.

### Es zeigen:

- Fig. 1: eine perspektivische Ansicht einer erfindungsgemässen Vorrichtung;
- Fig. 2: eine Seitenansicht der Vorrichtung nach Fig. 1; und
- Fig. 3: einen Längsschnitt durch eine modifizierte erfindungsgemässe Vorrichtung.

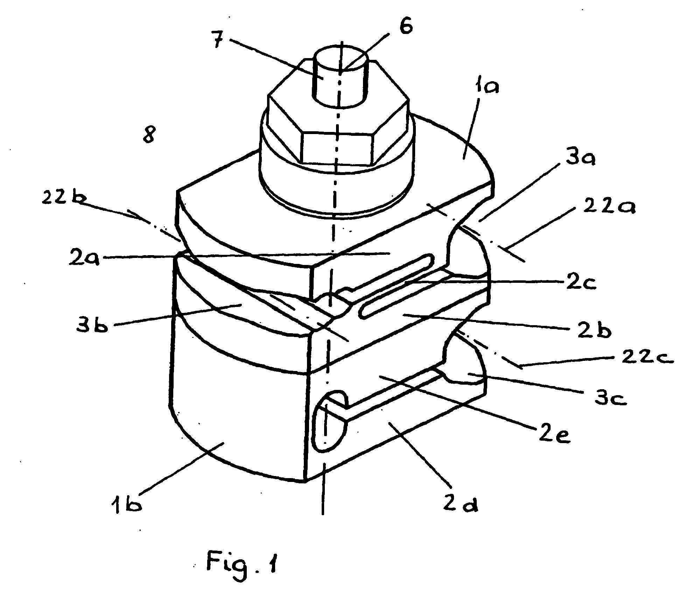

In den Fig. 1 bis 3 ist eine Ausführungsform dargestellt, welche zwei um eine Rotationsachse 6 gegeneinander verdrehbare Klammern 1 umfasst. Die zwei axial aneinander zur Anlage bringbaren Klammern 1 werden koaxial zur Rotationsachse 6 von einer Bohrung 4 durchdrungen, welche zur Aufnahme einer mit den Blockiermitteln 8 verbindbaren Welle 7 geeignet ist. Die Durchmesser der Bohrung 4 und der Welle 7 sind so ausgestaltet, dass die Welle 7 in der Bohrung 4 ein Spiel aufweist.

Die obere Klammer 1a ist S-förmig ausgebildet und umfasst drei quer zur Rotationsachse 6 angeordnete Klammerschenkel 2, wovon der axial aussenstehende Klammerschenkel 2a und der mittlere Klammerschenkel 2c sowie der zur unteren Klammer 1b benachbarte, innenliegende Klammerschenkel 2b und der mittlere Klammerschenkel 2c je eine dazwischenliegende Klammeröffnung 3a;3b zur Aufnahme eines Stabes 20 aufweisen. Die Klammeröffnungen 3a;3b sind kanalartig ausgebildet und weisen parallele, zur Rotationsachse 6 orthogonale Kanalachsen 22a;22b auf.

Die untere Klammer 1b umfasst einen axial aussenstehenden Klammerschenkel 2d sowie einen zur oberen Klammer 1a benachbarten innenliegenden Klammerschenkel 2e. Die untere Klammer 1b weist nur eine Klammeröffnung 3c zwischen den Klammerschenkeln 2d;2e auf. Diese Klammeröffnung 3c bildet ebenfalls einen zur Aufnahme eines Stabes 20 geeigneten Kanal mit einer zur Rotationsachse 6 orthogonalen Kanalachse 22c.

Jede der Klammeröffnungen 3a;3b;3c lässt sich durch elastische Deformation der entsprechenden Klammer 1a;1b erweitern oder verengen. Die Elastizität der Klammerschenkel 2 wird durch entsprechend dünne Ausbildung der je zwei Klammerschenkel 2 zusammenhaltenden Längsstege 13 der Klammern 1 erreicht. Ferner sind alle Klammeröffnungen 3a;3b;3c an ihren von der Rotationsachse 6 entfernten Öffnungen je eine Verengung 10 aufweisen. Peripher sind die Klammeröffnungen 3a;3b;3c mit Erweiterungen 11 zur einfacheren Einführung eines Stabes 20 in die betreffende Klammeröffnung 3a;3b;3c versehen.

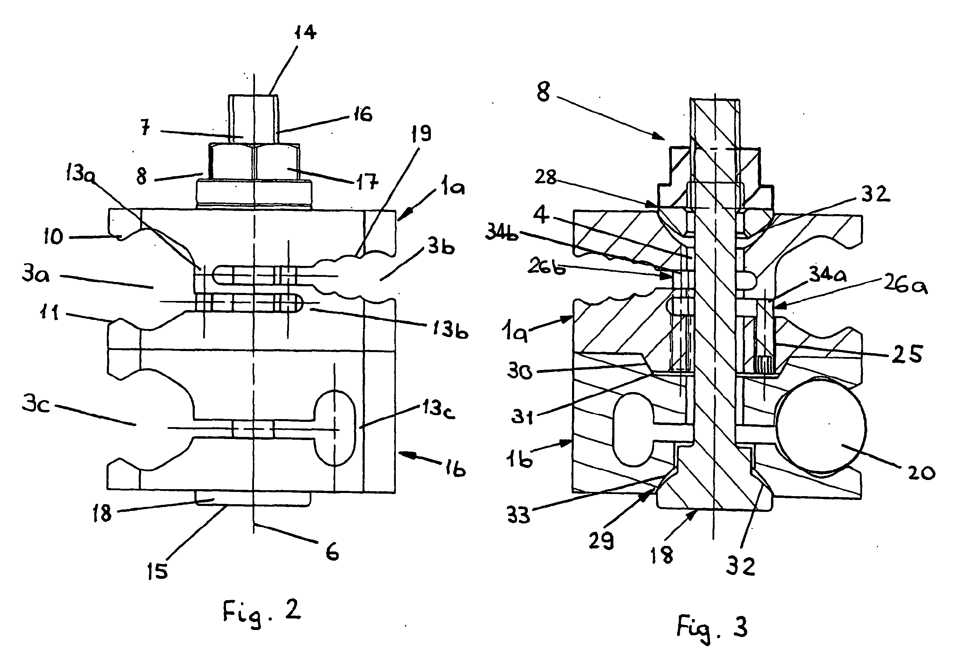

Wie in Fig. 2 gezeigt, weist die Welle 7 an ihrem ersten Ende 14 ein Aussengewinde 16 auf, worüber als Blockiermittel 8 eine Mutter 17 schraubbar ist. An ihrem zweiten Ende 15 ist die Welle 7 mit einem einen grösseren Durchmesser aufweisenden Kopf 18 versehen, welche am aussenstehenden Klammerschenkel 2d der unteren Klammer 1b aufliegt. Die Mutter 17 wird beim Anziehen gegen die äussere. Oberfläche des aussenstehenden Klammerschenkels 2a der oberen Klammer 1 a gepresst während der Kopf 18 am zweiten Ende 15 der Welle 7 gegen die äussere Oberfläche des aussenstehenden Klammerschenkels 2d der unteren Klammer 1b gepresst wird. Eine der Klammeröffnungen 3b in der oberen Klammer 1 a weist eine zu ihrer Kanalachse 22b orthogonalen Querschnittsfläche auf, deren Begrenzung an den Klammerschenkeln 2a;2c je drei Kreisbogen 19 mit unterschiedlichem Durchmesser aufweist, so dass in dieser Klammeröffnung 3b kreiszylindrische Stäbe 20 mit unterschiedlichen Durchmessern aufgenommen werden können.

Wie in Fig. 3 gezeigt, umfassen die einander benachbarten Klammerschenkel 2b;2e der beiden Klammern 1a; 1b ineinander eingreifend eine zur Rotationsachse 6 koaxiale, konische Erhebung 30, respektive zur Rotationsachse 6 koaxiale, konische Vertiefung 31 auf. Hier ist die konische Erhebung 30 an der an die untere Klammer 1 b angrenzenden Oberfläche der oberen Klammer 1 a angebracht während die dazu komplementäre konische Vertiefung 31 an der an die obere Klammer 1a angrenzenden Oberfläche der unteren Klammer 1b angebracht ist. Die miteinander in Kontakt stehenden Oberflächen der konischen Erhebung 30, respektive konischen Vertiefung 31 können aufgerauht sein, so dass eine relative Rotation der beiden Klammern 1a; 1b um die Rotationsachse 6 bei festgezogenen Blockiermitteln 8 verhindert wird. Ferner umfasst die, die beiden Klammern 1a; 1b durchdringende Bohrung 4 an den axial aussenstehenden Austritten Bohrungssegmente 29, welche je eine konkav kugelzonenartige Oberfläche 33 aufweisen. Zu diesen Bohrungssegmenten 29 komplementär ausgestaltet ist der Kopf 18 am zweiten Ende 15 der Welle 7 sowie ein koaxial zwischen der Mutter 17 und der oberen Klammer 1a angeordnetes Zwischenstück 28, d.h. der Kopf 18 weist gegen die untere Klammer 1b gerichtet eine konvex kugelzonenartige Oberfläche 32 auf und das Zwischenstück 28 weist gegen die obere Klammer 1a gerichtet ebenfalls eine konvex kugelzonenartige Oberfläche 32 auf.

Ebenfalls in Fig. 3 dargestellt sind die Arretierstifte 26 an der oberen Klammer 1 a. Diese Arretierstifte 26 sind mittels einer Gewindeverbindung in zur Rotationsachse 6 parallele Bohrungen 25 im innenliegenden Klammerschenkel 2b der oberen Klammer 1a einschraubbar und so justierbar, dass sie das Zusammenpressen der Klammeröffnungen 3a;3b an der oberen Klammer 1a begrenzen. Dazu werden die Arretierstifte 26 soweit eingeschraubt, bis das vordere Ende 34a des ersten Arretierstiftes 26a an der dem innenliegenden Klammerschenkel 2b benachbarten Oberfläche des mittleren Klammerschenkels 2c ansteht und das vordere Ende 34b des zweiten Arretierstiftes 26b an der dem mittleren Klammerschenkel 2c benachbarten Oberfläche des aussenstehenden Klammerschenkels 2a ansteht.

## Patentansprüche

1. Vorrichtung zur gegenseitigen Positionierung von longitudinalen Bauelementen, welche zwei um eine Rotationsachse (6) gegeneinander verdrehbare Klammern (1) umfasst, wobei
A) jede der beiden Klammern (1) mindestens zwei Klammerschenkel (2) umfasst;
B) je zwei Klammerschenkel (2) eine dazwischen liegende Klammeröffnung (3) definieren; wobei
C) die Klammeröffnung (3) durch elastische Deformation der Klammer (1) wahlweise verengbar oder erweiterbar ist, so dass ein in die Klammeröffnung (3) eingeführtes longitudinales Bauelement vorfixierbar ist;
D) beide Klammem (1) eine zur Rotationsachse (6) koaxiale Bohrung (4) aufweisen, welche die Klammerschenkel (2) durchquert;
E) die beiden Klammern (1) auf einer Welle (7) gelagert sind; und
F) Blockiermittel (8) vorgesehen sind, mittels welcher die Rotierbarkeit der beiden Klammern (1) um die Rotationsachse (6) wahlweise blockierbar ist, und welche dazu geeignet sind, die Klammerschenkel (2) jeder Klammer (1) gegen ein in die Klammeröffnung (3) eingeführtes longitudinales Bauelement zu pressen,
**dadurch gekennzeichnet, dass**
G) mindestens eine der beiden Klammern (1) S-förmig ausgebildet ist und zwei sich diametral gegenüberstehende Klammeröffnungen (3) definiert.

2. Vorrichtung nach Anspruch 1, **dadurch gekennzeichnet, dass** mindesten eine der beiden Klammern (1) eine oder mehrere parallel zur Rotationsachse (6) verlaufende Bohrungen (25) aufweist, in welchen Arretierstifte (26) parallel zur Rotationsachse (6) bewegbar sind.

3. Vorrichtung nach Anspruch 1 oder 2, **dadurch gekennzeichnet, dass** die Bohrung (4) in den beiden Klammern (1) mindestens teilweise einen grösseren Durchmesser als die Welle (7) aufweist.

4. Vorrichtung nach Anspruch 3, **dadurch gekennzeichnet, dass** die Klammerschenkel (2) derart miteinander verbunden sind, dass ein zangenähnliches Öffnen und Schliessen der Klammerschenkel (2) um einen Drehpunkt realisierbar ist.

5. Vorrichtung nach einem der Ansprüche 1 bis 4, **dadurch gekennzeichnet, dass** die Klammer (1) einstückig ausgebildet ist.

6. Vorrichtung nach einem der Ansprüche 1 bis 5, **dadurch gekennzeichnet, dass** die Klammeröffnung (3) eine Verengung (10) aufweist.

7. Vorrichtung nach einem der Ansprüche 1 bis 6, **dadurch gekennzeichnet, dass** die Klammeröffnung (3) nach Aussen eine Erweiterung (11) aufweist.

8. Vorrichtung nach einem der Ansprüche 1 bis 7, **dadurch gekennzeichnet, dass** die Klammeröffnungen (3) kanalartig ausgebildet sind und eine Kanalachse (22) aufweisen und dass mindestens eine der Klammeröffnungen (3) eine zu ihrer Kanalachse (22) orthogonale Querschnittsfläche aufweist, deren Begrenzung an den angrenzenden Klammerschenkeln (2) mehrere hintereinander angeordnete Kreisbogen (19) mit unterschiedlichen Durchmessern aufweist.

9. Vorrichtung nach einem der Ansprüche 1 bis 8, **dadurch gekennzeichnet, dass** die einander benachbarten Klammerschenkel (2) der beiden Klammern (1) ineinander eingreifend eine zur Rotationsachse (6) koaxiale konische Erhebung (30), respektive konische Vertiefung (31) umfassen.

10. Vorrichtung nach Anspruch 9, **dadurch gekennzeichnet, dass** die Oberflächen der konischen Erhebung (30) und/oder der konischen Vertiefung (31) aufgerauht sind.

11. Vorrichtung nach einem der Ansprüche 1 bis 10, **dadurch gekennzeichnet, dass** die Welle (7) in der Bohrung (4) ein Spiel aufweist.

12. Vorrichtung nach einem der Ansprüche 1 bis 11, **dadurch gekennzeichnet, dass** die Bohrung (4) an ihren aussenstehenden Austritten Bohrungssegmente (29) umfasst, welche je eine zur Rotationsachse (6) konzentrische, konkav kugelzonenartige Oberfläche (33) aufweist.

13. Vorrichtung nach Anspruch 12, **dadurch gekennzeichnet, dass** die Welle (7) einen einen grösseren Durchmesser aufweisenden Kopf (18) mit einer zur konkav kugelzonenförmigen Oberfläche (33) der Bohrungssegmente (29) komplementär konvex kugelzonenförmige Oberfläche (32) aufweist.

14. Vorrichtung nach Anspruch 12 oder 13, **dadurch gekennzeichnet, dass** die Blockiermittel (8) ein Zwischenstück (28) mit einer zur konkav kugelzonenförmigen Oberfläche (33) der Bohrungssegmente (29) komplementär konvex kugelzonenförmigen Oberfläche (32) aufweist.

15. Klammer (1) für eine Vorrichtung zur gegenseitigen Positionierung von longitudinalen Bauelementen,
**dadurch gekennzeichnet, dass**
A) die Klammer (1) S-förmig ausgebildet ist, so dass drei Klammerschenkel (2) und zwei sich diametral gegenüberstehende Klammeröffnungen (3) zur Aufnahme von longitudinalen Bauelementen definiert werden; wobei
B) die Klammeröffnungen (3) durch elastische Deformation der Klammer (1) wahlweise verengbar oder erweiterbar ist;
C) die Klammer (1) um eine Rotationsachse (6) drehbar ist, zu welcher sie eine koaxiale Bohrung (4) aufweist, welche die drei Klammerschenkel (2) durchquert; und
D) die Klammer (1) mittels ihrer Bohrung (4) auf einer Welle (7) lagerbar ist.

## Claims

1. Device for the mutual positioning of longitudinal components, which comprises two clamps (1), which can be rotated with respect to one another about an axis of rotation (6),
A) each of the two clamps (1) comprising at least two clamp limbs (2)
B) two clamp limbs (2) in each case defining a clamp opening (3) between said limbs (2),
C) the clamp opening (3) alternatively being constrictable or expandable, so that a longitudinal component, introduced into the clamp opening (3), can be prefixed,
D) both clamps (1) having a borehole (4), which is coaxial with the axis of rotation (6) and traverses the clamp limbs (2),
E) the two clamps (1) being mounted on a shaft (7) and
F) blocking means (8) being provided, by means of which the rotatability of the two clamps (1) about the axis of rotation (6) alternatively can be blocked and which are suitable for pressing the clamp limbs (2) of each clamp (1) against a longitudinal component, which has been introduced into the clamp opening (3),
**characterized in that**
G) at least one of the two clamps (1) is constructed S-shaped and defines two diametrically opposite clamp openings (3).

2. The device of claim 1, **characterized in that** at least one of the two clamps (1) has one or more boreholes (25), which extend parallel to the axis of rotation (6) and in which locking-in-position pins (26) can be moved parallel to the axis of rotation (6).

3. The device of claims 1 or 2, **characterized in that** the borehole (4) in a two clamps (1) has at least partially a diameter larger than that of the shaft (7).

4. The device of claim 3, **characterized in that** the clamp limbs (2) are connected with one another in such a manner, that a pliers-like opening and closing of the clamp limbs (2) about a fulcrum can be realized.

5. The device of one of the claims 1 to 4, **characterized in that** the clamp (1) is constructed in one piece.

6. The device of one of the claims 1 to 5, **characterized in that** the clamp opening (3) has a constriction (10).

7. The device of one of the claims 1 to 6, **characterized in that** the clamp opening (3) has an expansion (11) towards the outside.

8. The device of one of the claims 1 to 7, **characterized in that** the clamp openings (3) are constructed in the form of channels and have a channel axis (22) and that at least one of the clamp openings (3) has a cross sectional surface, which is orthogonal to its channel axis (22) and the boundary of which at the adjoining clamp limbs (2) has several circular arcs (19) of different diameters, disposed one behind the other.

9. The device of one of the claims 1 to 8, **characterized in that** the mutually adjacent clamp limbs (2) of the two clamps (1) comprise a conical elevation (30) and a conical depression (31) respectively, which are coaxial with the axis of rotation (6) and engage one another.

10. The device of claim 9, **characterized in that** the surfaces of the conical elevation (30) and/or of the conical depression (31) are roughened.

11. The device of one of the claims 1 to 10, **characterized in that** the shaft (7) has clearance in the borehole (4).

12. The device of one of the claims 1 to 11, **characterized in that** the borehole (4), at its outer outlets, comprises borehole segments (29), each of which has a concave, spherical zone-like surface (33), which is concentric with the axis of rotation (6).

13. The device of claim 12, **characterized in that** the shaft (7) has a head (18) of larger diameter with a convex spherical zone-like surface (32), which is complementary to the concave, spherical zone-like surface (33) of the borehole segments (29).

14. The device of claims 12 or 13, **characterized in that** the blocking means (8) has an intermediate piece (28) with a convex, spherical zone-like surface (32), which is complementary to the concave spherical zone-like surface (33) of the borehole segments (29).

15. Clamp (1) for a device for the mutual positioning of longitudinal components,
**characterized in that**
A) the clamp (1) is constructed S-shaped, so that the three clamp limbs (2) and two diametrically opposite clamp openings (3) are defined for accommodating longitudinal components,
B) the clamp openings (3) alternatively being constrictable or expandable by elastic the formation of the clamp (1),
C) the clamp (1) is rotatable about an axis of rotation (6) to which it exhibits a coaxial borehole (4), which is coaxial with the axis of rotation (6) and traverses the three clamp limbs (2) and
D) the clamp (1) being mountable on a shaft (7) by means of its borehole (4).

## Revendications

1. Dispositif pour le positionnement mutuel d'éléments longitudinaux, comprenant deux brides (1) pouvant tourner l'une par rapport à l'autre autour d'un axe de rotation (6), dans lequel
A) chacune des deux brides (1) comprend au moins deux branches de bride (2) ;
B) deux branches de bride (2) définissent ensemble une ouverture de bride (3) située entre elles ;
dans lequel
C) l'ouverture de bride (3) peut, au choix, être réduite ou agrandie par déformation élastique de la bride (1), si bien qu'un élément longitudinal inséré dans l'ouverture de bride (3) peut être préfixé ;
D) les deux brides (1) présentent un alésage (4), coaxial à l'axe de rotation (6), qui traverse les branches de bride (2) ;
E) les deux brides (1) sont logées sur un arbre (7) ; et
F) des moyens de blocage (8) sont prévus, lesquels permettent de bloquer comme souhaité la rotation des deux brides (1) autour de l'axe de rotation (6) et sont appropriés pour presser les branches de bride (2) de chaque bride (1) contre un élément longitudinal inséré dans l'ouverture de bride (3),
**caractérisé en ce que**
G) au moins une des deux brides (1) est réalisée en forme de S et définit deux ouvertures de bride (3) diamétralement opposées.

2. Dispositif selon la revendication 1, **caractérisé en ce qu'**au moins une des deux brides (1) présente un ou plusieurs alésages (25) s'étendant parallèlement à l'axe de rotation (6), dans lesquels des chevilles d'arrêt (26) sont mobiles parallèlement à l'axe de rotation (6).

3. Dispositif selon la revendication 1 ou 2, **caractérisé en ce que** l'alésage (4) dans les deux brides (1) présente au moins en partie un diamètre supérieur à celui de l'arbre (7).

4. Dispositif selon la revendication 3, **caractérisé en ce que** les branches de bride (2) sont reliées l'une à l'autre de telle manière qu'une ouverture et une fermeture, analogues à celles d'une pince, des branches de bride (2) autour d'un centre de rotation peut être réalisée.

5. Dispositif selon l'une quelconque des revendications 1 à 4, **caractérisé en ce que** la bride (1) est formée d'une seule pièce.

6. Dispositif selon l'une quelconque des revendications 1 à 5, **caractérisé en ce que** l'ouverture de bride (3) présente un rétrécissement (10).

7. Dispositif selon l'une quelconque des revendications 1 à 6, **caractérisé en ce que** l'ouverture de bride (3) présente un évasement (11) vers l'extérieur.

8. Dispositif selon l'une quelconque des revendications 1 à 7, **caractérisé en ce que** les ouvertures de bride (3) sont réalisées en forme de canal et présentent un axe de canal (22), et **en ce qu'**au moins une des ouvertures de bride (3) présente une surface de section transversale orthogonale à son axe de canal (22) dont la limite avec les branches de bride (2) adjacentes présente plusieurs arcs de cercle (19) de différents diamètres disposés les uns derrière les autres.

9. Dispositif selon l'une quelconque des revendications 1 à 8, **caractérisé en ce que** les branches de bride adjacentes (2) des deux brides (1) comprennent une proéminence conique (30) coaxiale à l'axe de rotation (6) et un évidement conique (31) correspondant qui s'insèrent l'un dans l'autre.

10. Dispositif selon la revendication 9, **caractérisé en ce que** les surfaces de la proéminence conique (30) et/ou de l'évidement conique (31) sont rugueuses.

11. Dispositif selon l'une quelconque des revendications 1 à 10, **caractérisé en ce que** l'arbre (7) présente un certain jeu dans l'alésage (4).

12. Dispositif selon l'une quelconque des revendications 1 à 11, **caractérisé en ce que** l'alésage (4) comprend, au niveau de ses sorties externes, des segments d'alésage (29) qui présentent chacun une surface en forme de segment sphérique concave (33) concentrique à l'axe de rotation (6).

13. Dispositif selon la revendication 12, **caractérisé en ce que** l'arbre (7) présente une tête (18) ayant un diamètre supérieur, comprenant une surface en forme de segment sphérique convexe (32) complémentaire à la surface en forme de segment sphérique concave (33) des segments d'alésage (29).

14. Dispositif selon la revendication 12 ou 13, **caractérisé en ce que** les moyens de blocage (8) présentent une pièce intermédiaire (28) comprenant une surface en forme de segment sphérique convexe (32) complémentaire à la surface en forme de segment sphérique concave (33) des segments d'alésage (29).

15. Bride (1) destinée à un dispositif pour le positionnement mutuel d'éléments longitudinaux,
**caractérisée en ce que**
A) la bride (1) est réalisée en forme de S, de sorte que trois branches de bride (2) et deux ouvertures de bride (3) diamétralement opposées sont définies pour loger des éléments longitudinaux ;
dans laquelle
B) les ouvertures de bride (3) peuvent, au choix, être réduites ou agrandies par déformation élastique de la bride (1) ;
C) la bride (1) présente un alésage (4), coaxial à l'axe de rotation (6), qui traverse les trois branches de bride (2) ; et
D) la bride (1) peut être logée sur un arbre (7) au moyen de son alésage (4).
